(19) Europäisches Patentamt
European Patent Office
Office européen des brevets

(11) Numéro de publication: **0 128 097**
**B1**

(12) # FASCICULE DE BREVET EUROPÉEN

(45) Date de publication du fascicule du brevet: **13.01.88**

(21) Numéro de dépôt: **84401127.0**

(22) Date de dépôt: **04.06.84**

(51) Int. Cl.⁴: **C 07 K 9/00**, C 08 B 37/00, A 61 K 45/05, A 61 K 37/02, A 61 K 37/64, A 61 K 39/395

(54) **Nouveaux dérivés immunostimulants, leur préparation et leur application comme médicament.**

(30) Priorité: **03.06.83 FR 8309325**

(43) Date de publication de la demande: **12.12.84 Bulletin 84/50**

(45) Mention de la délivrance du brevet: **13.01.88 Bulletin 88/2**

(84) Etats contractants désignés: **AT BE CH DE FR GB IT LI LU NL SE**

(56) Documents cité:
**EP-A-0 013 651**
**EP-A-0 074 279**
**FR-A-2 421 178**
**FR-A-2 428 050**

**CHEMICAL ABSTRACTS, vol. 93, no. 19, 10 novembre 1980, page 474, no. 184067b, Columbus, Ohio, US; C.M. REICHERT et al.: "Synthesis of conjugates containing N-acetylmuramyl-L-alanyl-D-isoglutaminyl (MDP). Their use as hapten-carrier systems"**

(73) Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Monsigny, Michel, 341, rue des Bouvreuils, F-45590 Saint- Cyr- en- Val (FR)**
Inventeur: **Roche, Annie- Claude, 360, route de Saint- Mesmin, F-45750 Saint- Pryve (FR)**

(74) Mandataire: **Nony, Michel, Cabinet Nony 29, rue Cambacérès, F-75008 Paris (FR)**

**EP 0 128 097 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention a pour objet de nouveaux dérivés immunostimulants, leur préparation et leur utilisation comme médicament.

On sait que de nombreux extraits de mycobactéries, tels que la fraction soluble de la paroi (WSA), ainsi que leurs analogues synthétiques, tels que le muramyldipeptide (MDP), ont des activités adjuvantes comparables à celles des mycobactéries elles-mêmes dans l'adjuvant complet de Freund. Le MDP est particulièrement intéressant, car il peut être obtenu par synthèse (C. Merser et al., 1975, Biochem. Biophys. Res. Commun. 66, 1316-1322) et de ce fait il est disponible en quantité non limitée; en outre, il a une faible masse moléculaire (492) et il n'est pas toxique. Les propriétés biologiques du MDP et de ses nombreux dérivés ont trait à l'activité adjuvante, à la stimulation générale du système immun et à la résistance aux infections (voir revues: Adam, A. et al., 1981, Mol. Cell. Biochem. 41, 27-47; Leclerc, C. and Chedid, L., 1982, in Lymphokines E. Pick ed., Academic Press, vol. 7, ppl-21.

Il est connu que le muramylpeptide agit in vitro sur des cellules du système réticuloendothélial (macrophages) en augmentant leur activité phagocytique (Tanaka et al., 1977, Biochem. Biophys. Res. Commun. 77, 621-627).

Le MDP et les divers autres extraits mycobactériens augmentent également l'activité cytotoxique et cytolytique des macrophages péritonéaux de souris in vitro (Juy, C. and Chedid, L., 1975, Proc. Nat. Acad. Sci. 72, 4105-4109). Si le MDP, à des concentrations relativement faibles (10 µg/ml), entraîne un effet de cytostaticité des macrophages sur les cellules tumorales in vitro, son utilisation in vivo n'est pas possible directement car il est excrété très rapidement dans l'urine (50 % en 30 min. et plus de 90 % en 2 h) chez la souris (Parant et al., 1979 Int. J. Immunopharmacol. 1, 35-41). Afin d'augmenter la durée de vie du MDP dans l'organisme, divers oligomères et polymères ont été préparés, non spécifiques de cellules cibles, dont quelques une présentent une certaine activité. En particulier, Chedid, L. et al., (1979, Proc. Nat. Acad. Sci. 76 6557-6561) ont montré que le MDP lié à un multipolymère (multipoly-D-L-alanine-poly-L-lysine) induit une résistance accrue à l'infection mais aussi un effet pyrogénique important.

La présente invention a pour objet de nouveaux conjugués macromoléculaires de muramylpeptides et analogues. Ces dérivés sont des immunostimulants dont certains non seulement sont plus actifs que les immunostimulants libres (MDP, dérivés et analogues) pour induire la tumoricidie des macrophages in vitro, mais encore éradiquent les métastases spontanées d'un carcinome pulmonaire de souris, in vivo alors que les immunostimulants libres sont inefficaces.

La présente invention a plus précisèment pour objet les nouveaux dérivés de formule générale I:

$(R\text{-}X)_m$ - (LAM) I

dans laquelle R est le résidu d'un immunostimulant du type N-acétylmuramyl-peptide ou d'un de ses dérivés ou analogues,

(LAM) est un ligand affine des macrophages ou des monocytes, ou un précurseur d'un tel ligand,

et X représente soit une liaison covalente entre R et (LAM) soit le résidu d'un agent de couplage permettant de fixer R sur le ligand (LAM),

et m est un nombre supérieur ou égal à 1.

La limite supérieure du nombre m est variable et est déterminée dans chaque cas par des expériences simples, comme cela sera exposé ci-après.

Parmi les composés de formule, on citera en particulier:

A) ceux pour lesquels R représente le résidu d'un composé comportant la sequence N-acétylmuramyl-L-Ala-, en particulier la séquence:

N- acétylmuramyl-L-Ala-D-iso-Gln-

ou N-acétylmuramyl-L-Ala-D-Glu-.

R est par exemple le résidu d'un des dérivés de MDP mentionnés dans la revue de ADAM A. et al, 1981, Mol. Cell. Biochem. 41, 24-47, et en particulier R peut représenter le résidu:

- du MDP (N-acétylmuramyl-L-Ala-D-isoGln)
- du MTP (N-acétylmuramyl-L-Ala-D-isoGln-meso-$A_2$pm)
- du MQF (N-acétylmuramyl-L-Ala-D-isoGln-meso-$A_2$pm-D-Ala)
- du MPP (N-acétylmuramyl-L-Ala-D-isoGln-meso-$A_2$ pm-D-Ala-D-Ala)

ou un résidu muramylpeptidique substitué par un amino-acide, par un peptide, par un groupement bifonctionnel, par la glutaraldéhyde ou par l'acide p-thiocarbamyl benzoïque.

Dans ce qui précède, Ala- est le symbole du groupement alanyle; meso-$A_2$ pm représente le groupement mesodiaminopimélyle et isoGln- le groupement isoglutaminyle;

B) Ceux pour lesquels (LAM) représente:

1) les glycoconjugués reconnus sélectivement par les lectines membranaires des macrophages ou des monocytes et en particulier:

a) les glycoprotéines naturelles ou modifiées portant des structures glucidiques contenant par exemple des résidus α-D-mannose, 6-phosphoryl α-D-mannose, ou β-N-acétylglucosamine; les glycoprotéines naturelles sont décrites par exemple dans l'ouvrage de Jean MONTREUIL, "Glycoproteins", in Comprehensive Biochemistry, vol. 19, B2, Albert Neuberger Ed. (Elsevier) 1982.

b) les oligosaccharides ou les glycopeptides contenant l'un au moins des résidus α-D-glucopyranoside, α-D-mannopyranoside, α-L-fucopyranoside, (α ou β)-L-galactopyranoside, α-D-galactopyranoside, 2-acétamido-2-

déoxy-(α ou β)-D-glucopyranoside, 2-acétamido-2-déoxy-α-D-galactopyranoside, 2-acétamido-2-déoxy-β-D-galactopyranoside, 6-phosphoryl-α-D-mannopyranoside, ou α-L-rhamnopyranoside. Les oligosaccharides utilisés selon la présente invention sont notamment les oligosaccharides libérés des glycoprotéines naturelles par des endoglycosidases, ou encore les analogues synthétiques de ces oligosaccharides.

On sait que pour pouvoir être reconnus par les récepteurs membranaires, les oligosaccharides doivent contenir au moins 5 motifs osidiques. Pour cette raison, on utilise selon la présente invention des oligosaccharides et oligopeptides comportant au moins 5 motifs osidiques, par exemple des oligosaccharides ou oligopeptides comportant de 5 à 18 motifs osidiques. Les glycopeptides utilisés selon l'invention sont notamment les glycopeptides issus des glycoprotéines naturelles. Ces glycopeptides sont décrits notamment par J. MONTREUIL (ouvrage cité). Ils peuvent être préparés par traitement enzymatique des glycoprotéines naturelles selon les méthodes décrites par exemple par MONSIGNY et al. Bull. Soc. Chim. Biol. Vol. 50, pp 857-886; ou bien on utilise des glycopeptides synthétiques ou semi-synthétiques analogues aux glycopeptides obtenus selon ledit traitement enzymatique des glycoprotéines naturelles. Par convention, on appelle ici "glycopeptide" un dérivé constitué par une chaîne glucidique liée à au moins un acide aminé qui est notamment l'asparagine, la sérine ou la thréonine pour des raisons qui apparaîtront ci-après.

Les motifs saccharidiques des oligosaccharides et/ou des glycopeptides utilisés selon l'invention peuvent être partiellement estérifiés, par exemple sous forme de phosphates.

c) les néoglycoprotéines, c'est-à-dire des protéines substituées par des copules glycidiques.

Les néoglycoprotéines sont obtenues notamment selon l'ouvrage de LEE Y. C. and LEE R.T., 1982, in the Glycoconjugates, M.I. HOROWITZ ED., Academic Press, Vol.4, pages 57-83.

Les protéines de départ sont de préférence des protéines syngéniques, par exemple le sérumalbumine, le lysozyme, la ferritine ou une immunoglobuline.

On sait que dans les glycoprotéines naturelles, les chaînes glucidiques sont combinées à um acide aminé de la protéine: soit à l'asparagine par une liaison amide, soit à un acide aminé alcool (sérine, thréonine, hydroxyproline) par une liaison osidique. Ces liaisons sont représentées schématiquement par les formules partielles suivantes:

- liaison avec l'asparagine:

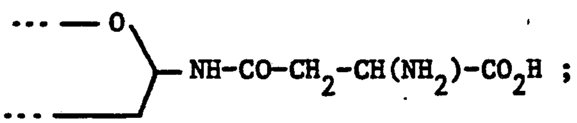

- liaison avec un acide aminé alcool (cas de la sérine):

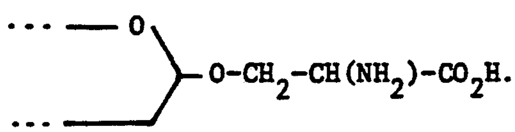

Bien entendu, dans les néoglycoprotéines, les chaînes glucidiques peuvent être reliées aux chaînes peptidiques par l'intermédiaire des mêmes aminoacides. Dans ces néoglycoprotéines, chaque chaîne glucidique introduite comporte de préférence au moins 5 motifs osidiques.

Le nombre minimum de copules glycidiques fixées sur la protéine est déterminé expérimentalement dans chaque cas par le seuil d'apparition d'une affinité de la néoglycoprotéine pour les macrophages ou les monocytes, cette affinité devant être suffisante pour permettre une endocytose efficace (par exemple lorsque la néoglycoprotéine est utilisée à une concentration inférieure à une micromole/litre).

Ce nombre minimum est généralement supérieur à 5.

Les spécialistes comprendront qu'il n'y a pas, en général, pour ce qui concerne le nombre maximum de copules glycidiques fixées, de seuil maximum autre que celui correspondant à la saturation des sites réactifs de fixation sur la protéine.

Les copules glucidiques sont choisies notamment parmi les osides de l'α-D-glucose, de l'α-D-mannose, de l'α-L-fucose, de la N-acétylglucosamine, de l'α-L-glucose, du β-L-galactose, du β-D-galactose, de l'α-D-galactosamine, de la β-D-galactosamine, de l'α-L-rhamnose ou du 6-phosphoryl-α-D-mannose, et comportent un groupement permettant l'établissement d'une liaison covalente avec la protéine (par exemple un groupement isothiocyanate, un groupement diazophényle ou un groupement thioéthanoyle).

Pour préparer des néoglycoprotéines, on peut utiliser par exemple une méthode anallogue à celle décrite par Mc BROOM et al, Met. Enzymol., 28, 212-219 (1972) qui consiste à préparer un dérivé nitrophénylé d'un saccharide et à réduire le groupement nitrophényl en phényl-amine primaire. On peut alors transformer le

groupement amine primaire en groupement isothiocyanate puis fixer le produit obtenu sur la protéine choisie, cette fixation se faisant par réaction du phényl isothiocyanate avec les groupements aminés de la protéine, avec formation d'un groupement phénylthiocarbamyle. On peut aussi transformer le dérivé à groupement phénylamine primaire en sel de diazonium correspondant. On peut alors fixer le produit obtenu sur la protéine choisie, cette fixation se faisant par formation d'un dérivé azoïque avec certains aminoacides de la protéine, qui contiennent des groupements aromatiques activés (notamment tyrosine, tryptophane), ou encore par réaction comportant un groupement aminé comme dans la lysine, un groupement guanidine comme dans l'arginine ou un groupement imidazole comme dans l'histidine.

Parmi les néoglycoprotéines on peut citer en particulier celles qui comportent des osides choisis parmi:

- un résidu thiocarbamylphényl-α-D-glucopyranoside
- un résidu thiocarbmylphényl-β-D-galactopyranoside
- un résidu thiocarbmylphényl-α-D-mannopyranoside
- un résidu thiocarbamylphényl-2-acétamido-2-déoxy-β-D-glucopyranoside
- un résidu thiocarbamylphényl-α-L-fucopyranoside
- un résidu thiocarbamylphényl-α-D-galactopyranoside
- un résidu thiocarbamylphényl-α-L-rhamnopyranoside
- un résidu thiocarbamylphényl-6-phosphoryl α-D-mannopyranoside
- un résidu thiocarbamylphényl-2-acétmido-2-déoxy-α-D-glucopyranoside
- un résidu thiocarbamylphényl-2-acétamido-2-déoxy-α-D-galactopyranoside
- un résidu thiocarbamylphényl-2-acétamido-2-déoxy-β-D-galactopyranoside
- un résidu thiocarbamylphényl-β-D-galacto-pyranosyl-4-β-D-glucopyranoside
- un résidu azophényl-β-D-galactopyranosyl-4-β-D-glucopyranoside
- un résidu azophényl-α-D-glucopyranoside
- un résidu azophényl-α-D-mannopyranoside
- un résidu azophényl-α-L-fucopyranoside
- un résidu azophényl-α-D-galactopyranoside
- un résidu azophényl-2-acétamido-2-déoxy-β-D-glycopyranoside
- les résidus d'oligosaccharides ou de glycopeptides renfermant l'un au moins des résidus α-D-glucopyranoside, α-L-glucopyranoside, α-D-mannopyranoside, α-L-fucopyranoside, β-L-galactopyraoside, β-D-galactopyranoside ou 2-acétamido-2-déoxy-β-D-glucopyranoside, 2-acétamido-2-déoxy-α-D-galactopyranoside, 2-acétamido-2-déoxy-β-D-galactopyranoside, 6-phosphoryl-α-D-mannopyranoside, α-L-rhamnopyranoside.

d) les polysaccharides naturel ou modifiés portant des résidus α-D-glucose, α-D-mannose, α-L-fucose ou N-acétylglucosamine.

Parmi ces polysaccharides, on citera par exemple des polysaccharides de levures (notamment le mannane), des polysaccharides de bactéries (notamment d'Escherichia coli, de Salmonella, de Klebsiella) ou d'autres polysaccharides (fucoïdine, lentinane).

2) des protéines modifiées par des substitutions abaissant le point isoélectrique, soit par blocage des groupements aimne libres, soit par introduction de groupements carboxyliques, soit encore par l'une ou l'autre méthode (voir par exemple Science, vol 218, 1982, p. 574-576).

Parmi ces protéines modifiées on peut citer les protéines N-acylées (telles que des protéines succinylées, maléylées, citraconylées, aconitylées, acétylées ou les protéines S-carboxyméthylées etc...

Pour ces modifications des protéines, on peut citer Methods in Enzymology, vol XI et vol XXV (Academic Press).

Les protéines de départ sont de préférence des protéines syngéniques;

3) des antiprotéases telles que 1'$\alpha_2$-macroglobuline, 1'$\alpha_1$-anti-trypsine, et plus généralement les antiprotéases sériques syngéniques qui sont connues et décrites par exemple dans Methods in Enzymology, vol 80 (Academic Press).

Ces antiprotéases doivent être considérées comme des précurseurs de ligand affine des macrophages car elles forment spontanément des complexes avec de nombreuses protéases (et en particulier les protéases secrétées par les cellules tumorales). Or il est connu que ces complexes protéase-antiprotéase ont une affinité pour les macrophages ou les monocytes; voir par exemple Jerry KAPLLAN et al, The Journal of Biological Chemistry, vol 254, N° 15, 7329-7335 (1979);

4) les anticorps (en particulier les anticorps monoclonaux) anticellules tumorales qui formeront dans l'organisme des complexes avec les antigènes solubles ou membranaires de ces cellules.

Il est bien connu que ces complexes sont sélectivement reconnus par les macrophages.

Ces anticorps constituent donc des précurseurs de ligand affine de macrophages;

C) Ceux pour lesquels X représente une liaison covalente entre un groupement fonctionnel de la partie peptidique de R et un groupement fonctionnel de (LAM); ou ceux pour lesquels X est le résidu d'un agent de couplage usuel (molécule bifonctionnelle) permettant de coupler R avec (LAM) par l'établissement de liaisons covalentes.

L'invention à également pour objet le procédé de préparation des composés de formule I.

Ce procédé est caractérisé par le fait que l'on fait réagir, selon les méthodes connues, le dérivé (R) correspondant au résidu R avec le ligand (LAM), éventuellement en présence d'un agent de couplage.

En variante, on peut faire réagir soit (R) soit (LAM) avec une des fonctions réactives de l'agent de couplage

(l'autre fonction étant éventuellement temporairement bloquée) puis l'on fait réagir le produit obtenu, du type RX ou $(X)_m$ - (LAM), avec (LAM) ou (R) respectivement.

En ce qui concerne le nombre m de molécules (R) qu'il convient de faire réagir avec la molécule (LAM), ce nombre est, comme indiqué précédemment, au moins égal à 1.

La limite supérieure de ce nombre m est déterminée dans chaque cas:

a) par l'observation da la parte du phénomène de reconnaissance sélective du LAM substitué vis-à-vis des récepteurs du macrophage (en raison de la saturation des sites de reconnaissance de la molécule LAM par les substituants RX-); ou

b) de même, dans le cas où LAM est un anticorps, par la perte de la capacité du LAM substitué à de fixer sur l'antigène correspondant; ou c) de même, dans le cas où LAM est une antiprotéase, par la perte de la capacité du LAM substitué de fixer les protéases. Cette limite supérieure de m peut donc être facilement déterminée par de simples expériences de routine in vitro dans chaque cas.

D'une façon générale, on peut faire réagir lea groupements amine (lorsqu'ils sont présents) du dérivé de MDP avec les groupements carboxliques (sous forme activée) du ligand (LAM). Bien entendu, il est possible de faire apparaître sur le ligand (LAM) des groupements carboxyliques ou d'en augmenter le nombre. Par exemple, si le ligand (LAM) est une protéine, une glycoprotéine ou une néoglycoprotéine, il est possible de faire apparaître des groupements carboxyliques par l'action d'un dérivé de diacide carboxylique, par exemple par succinylation. Les groupements carboxyliques libres du dérivé obtenu sont alors convenablement activés pour réagir avec les groupements $NH_2$ du dérivé de MDP qui est ainsi fixé avec formation d'une liaison amide.

De façon inverse, il est possible de faire réagir les groupements aminés du ligand (LAM) avec les groupements carboxyliques du dérivé de MDP (par exemple le groupement carboxylique de l'iso-Gln). Ici encore, il est possible de faire apparaître des groupements aminés supplémentaires sur le ligand; par exemple, si le ligand est une protéine, une glycoprotéine ou une néoglycoprotéine, on peut la succinyler puis faire réagir le dérivé succinylé avec une diamine, selon une méthode analogue à celle décrite ci-dessus. On peut aussi former, par l'action de l'hydrazine, les hydrazides des acides aminés du ligand.

Lorsque le ligand (LAM) est une protéine N-acylée, on peut aussi fixer la protéine au dérivé de MDP avant l'acylation de la protéine. On donne ci-après quelques exemples illustrant, à titre non limitatif, la fixation du ligand (LAM) sur le dérivé de MDP. Par exemple, pour fixer un oligosaccharide ou un polysaccharide sur le MDP (ou sur un de ses dérivés) on peut introduire les groupements amine primaire dans le saccharide en faisant réagir celui-ci avec un aminoéthylsulfate; ou bien on fait réagir le saccharide avec un bromoacétate, puis l'ester acétique obtenu avec une diamine (par exemple éthylènediamine) de façon à obtenir un aminoéthyl- monoamide correspondant. On peut aussi introduire un groupement amine sur le polysaccharide en estérifiant des groupements alcool du polysaccharide avec le carbonyldiimidazole, puis en faisant réagir le produit obtenu avec une diamine pour obtenir comme précédemment un dérivé aminé correspondant du polysaccharide. Par exemple avec l'éthylénediamine, on obtient un dérivé du type Sacch. $-O-CO-NH-(CH_2)-NH_2$ (Sacch. étant le reste du polysaccharide). On peut alors faire réagir le dérivé aminé obtenu pour former une liaison amide avec un groupement carboxyle du dérivé de MDP (par exemple le groupement carboxyle de l'iso-Gln), ledit groupement carboxyle étant bien entendu activé selon les méthodes usuelles (par exemple sous la forme d'un ester d'hydroxysuccinimide). Alternativement, la technique au bromoacétate permet de faire apparaître sur le saccharide un groupement carboxylique qui réagira avec les groupements amine du dérivé et permettra donc la fixation du saccharide sur le dérivé de MDP par l'établissement d'une liaison amide.

Bien entendu, les méthodes décrites ci-dessus pour les oligosaccharides sont applicables aux glycopeptides. On peut également introduire un groupement carboxylique sur un saccharide (ou oligo-saccharide ou polysaccharide) sous la forme d'un dérivé 1-déoxy 1-carboxyalkylthio pyranoside.

Par exemple, au départ du 1-chloro pyranoside correspondant (dont les groupements hydroxyle sont convenablement protégés sous forme de benzoates) on obtient, par l'action de la thiorurée, le chlorure de 1-isothiouronium correspondant. Par l'action du bromoacétate de méthyle, en présence de dithionite de sodium, on obtient le dérivé 1-thioéthianoate de méthyle correspondant qui peut être débenzoylé. Cet ester méthylique peut ensuite être saponifié en dérivé carboxylique correspondant et le groupement carboxylique est activé (par exemple par formation d'ester d'hydroxysuccinimide). Le dérivé thioéthanoïque activé obtenu pourra réagir par exemple avec les groupemants aminés (s'ils sont présents) du dérivé de MDP.

Un procédé analogue de thioéthanoylation d'un dérivé saccharidique est décrit par exemple dans la demande de brevet FR-A-2 551 442 déposée le 5 septembre 1983, intitulée "Nouveaux dérivés furaniques, leur préparation et leur application".

On peut aussi faire apparaître des groupements $-NH_2$ sur les oligosaccharides et glycopeptides contenant des motifs N-acétyl osamine (par exemple N-acétylglucosamine), par hydrazinolyse.

Pour fixer le dérivé de MDP sur un anticorps, on peut par exemple utiliser un agent de couplage bifonctionnel, ou bien faire réagir l'anticorps avec un ester activé du dérivé de MDP, (fixation par établissement d'une liaison amide avec les groupements $NH_2$ de l'anticorps), etc...

Come indiqué ci-dessus, les composés de formule 1 présentent des propriétés pharmacologiques intéressantes. Ces propriétés, jointes à une toxicité réduite, permettant leur utilisation comme médicaments.

L'invention a donc également pour objet l'utilisation comme médicaments des dérivés de formule I.

Les médicaments de l'invention sont des activateurs du système cellulaire immun induisant une protection et une thérapeutique notamment contre les infections bactériennes et le développement des cellules tumorales, et en particulier des cellules de métastases spontanées.

L'invention s'étend aux compositions pharmaceutiques contenant au moins un médicament de formule I. Ces compositions sont préparées selon les méthodes usuelles.

Ces compositions pharmaceutiques peuvent être administrées notamment par injection intra-veineuse, intra-tumorale ou encore par voie aérienne.

A cet effet, elles peuvent être présentées sous la forme de solutions ou suspensions injectables, ou sous forme de solutions ou suspensions pour aérosols.

La posologie varie avec la voie d'administration, le poids corporel et l'affection traitée. Par exemple, par voie intra-veineuse, elle est de l'ordre de 50 microgrammes à 500 microgrammes (en poids de MDP ou analogue) par kg.

Les exemples suivants illustrent l'invention sans la limiter.

**EXEMPLE 1**

Néoglycoprotéine du type (α-Man-PTC)$_{25}$-Sérum albumine-(MDP)$_{20}$.

a) La sérum albumine bovine (MM 67000; 50 mg) est dissoute dans 5 ml de tampon carbonate de sodium 0.10 M de pH 9.3 à 4°C. A la solution, on ajoute 40 mg de O-phénylisothiocyanate α-D-mannopyranoside (préparé selon McBroom et al., 1972 Met. Enzymol. 28, 212-219). Après 10h d'agitation à 4°C, la néoglycoprotéine est purifiée par tamisage moléculaire sur une colonne d'Ultrogel GF05 (3x40 cm), les fractions contenant la néoglycoprotéine sont dialysées contre de l'eau distillée puis lyophilisées.

b) Le muramyldipeptide (N-acétylmuramyl-L-alanyl-D-isoglutamine) (30 mg, 60 µmoles) est dissous dans 0.6 ml de N-diméthylformamide fraîchement distillé sur fluorodinitro benzène. On ajoute à la solution maintenue à 4°C, 1,2 équivalents de dicyclohexyl carbodiimide (15 mg, 72 µmoles) et 1,2 équivalent de N-hydroxysuccinimide (8.3 mg; 7294 moles). La solution est agitée 24 h. L'avancement de la réaction est suivi en chromatographie en couche mince de gel de silice (mélange solvant: chloroforme:méthanol:acide acétique: eau, 2:2:1:1, vol/vol). Rf du MDP:0,57, Rf du MDP-O-Su: 0,67. Le précipité de cyclohexylurée est éliminé par centrifugation (10 min 2000 g) ou par filtration sur verre fritté.

Note: MDP-O-Su signifie: ester d'hydroxysuccinimide du MDP.

c) La solution de muramyldipeptide activé (MDP-O-Su) (60µl contenant 3 mg de MDP) est ajoutée à une solution de néoglycoprotéine (Man)$_{25}$-SAB (5 mg dans 150 µl d'acétate de sodium 1M, pH 8,5). Le mélange réactionnel est agité pendant 24 h à 25°C. L'immunostimulant (Man)$_{25}$-SAB-(MDP)$_{10}$ est purifié par tamisage moléculaire sur une colonne d'Ultrogel GF05 dans du sérum physiologique tamponné. Les fractions renfermant l'immunostimulant (Man)$_{25}$-SAB-(MDP)$_{10}$ sont regroupées puis la solution est stérilisée par filtration sur Millipore (0,2 µm) et conservée à -20°C. Alternativement, la solution peut être dialysée puis lyophilisée.

Note: PTC signifie: phénylthiocarbamyl. On a aussi employé la notation (α-Man)$_{25}$ en omettant la mention du résidu de l'agent de couplage "PTC" qui est alors sous-entendue. SAB signifie: sérum albumine bovine.

**EXEMPLE 2**

Néoglycoprotéine du type (α-Man)$_{25}$-sérum albumine-(MDP)$_{20}$. Cet immunostimulant est préparé de façon identique à celui de l'exemple 1 sauf que la quantité du MDP-O-Su ajoutée dans le stade 1-c est de 6 mg en MDP dans 60 microlitres de diméthylformamide.

Remarque: Bien entendu, (α-Man) représente ici, en fait, (α-Man-PTC).

**EXEMPLE 3**

Néoglycoprotéine du type (α-Glc)$_{28}$-Sérum albumine-(MDP)$_{20}$.

Cet immunostimulant est préparé de façon identique à celui de l'exemple 1 sauf que l'O-phénylisothiocyanate d'α-D-glucopyranoside est utilisé à la place du dérivé α-D-mannopyranoside.

Remarque: (α-Glc) représente ici, plus exactement, (α-Glc-PTC).

**EXEMPLE 4**

Néoglycoprotéine du type (α-Fuc)$_{32}$-Sérum albumine-(MDP)$_{10}$.

On opère comme à l'exemple 3, mais avec l'O-phénylisothiocyanate d'α-L-fucopyranoside. Ici encore, (α-Fuc) est une notation abrégée pour (α-Fuc-PTC).

**EXEMPLE 5**

Néoglycoprotéine du type (β-GlcNAc)$_{25}$-Sérum albumine-(MDP)$_{10}$.

On opère comme à l'exemple 3, mais avec l'O-phénylisothiocyanate de 2-acétamido-2-déoxy-β-D-glycopyranoside.

Remarque: (β-GlcNAc) représente plus précisément (β-GlcNAc-PTC).

**EXEMPLE 6**

Néoglycoprotéine du type (β-Gal)-Sérum albumine-(MDP)$_{10}$.

On opère comme à l'exemple 3, mais avec l'O-phénylisothiocyanate de β-galactopyranoside. Pour la notation, voir les remarques dans les exemples précédents.

**EXEMPLE 7**

Néoglycoprotéine du type (β-Lac)$_{28}$-Sérum albumine-(MDP)$_{10}$.

On opère comme à l'exemple 3 mais avec l'O-phénylisothiocyanate de β-lactopyranoside. Pour la notation, voir les remarques dans les exemples précédents.

**EXEMPLE 8**

Néoglycoprotéine du type (di-N-acétylchitobioside)$_{20}$-Sérum albumine-(MDP)$_{10}$.

On opère comme à l'exemple 3 mais avec l'O-phénylisothiocyanate de 2-acétamido-2-déoxy-4 (1-acétamido-2-déoxy-β-D-glycopyranosyl)-β-D-glycopyranoside. Bien entendu, (di-N-acétylchitobioside) est une notation abrégée pour (di-N-acétylchitobioside-PTC).

**EXEMPLE 9**

Néoglycoprotéine du type (α-Man)$_{20}$-Sérum albumine-(MDP)$_{10}$.

a) la néoglycoprotéine est préparée par action du chlorydrate d'O-diazophényl-α-D-mannopyranoside dans les conditions précédemment décrites (FEBS Letters, 1977, 76, 257-261).

Les étapes b) et c) sont identiques à celles décrites en 1b et 1c.

Remarque: Bien entendu, (α-Man) est ici une notation abrégée pour (azophényl-α-Man).

**EXEMPLE 10**

Néoglycoprotéine du type (α-Man)$_{25}$-Sérum albumine-(MPP)$_{10}$.

a) la néoglycoprotéine est préparée selon la méthode décrite en 1a. (α-Man) signifie donc ici (α-Man-PTC). Ensuite la néoglycoprotéine (20 mg) est dissoute dans 5 ml d'acétate de sodium saturé. A cette solution, on ajoute 10 mg d'anhydride succinique. Après 12 h d'agitation la néoglycoprotéine succinylée est dialysée contre de l'eau distillée puis lyophilisée.

b) la néoglycoprotéine succinylée est dissoute dans 10 ml de N-diméthylformamide anhydre. A cette solution on ajoute du dicyclohexylcarbodiimide (5 mg, 24 μmoles) puis de l'hydroxysuccinimide (2,8 mg; 24 μmoles) à 4°C. Après 24 h d'agitation à température ambiante, on ajoute 5 mg de N-acétylmuramyl pentapeptide dans 5 ml de tampon carbonate de sodium 0,1M. Le mélange réactionnel est agité 18 h à 4°C. L'immunostimulant (α-Man)$_{25}$-Sérum albumine-(MPP)$_{10}$ est purifié par chromatographie de tamisage moléculaire dans une colonne d'Ultrogel AcA 202 stabilisée dans un tampon PBS pH 8,5. L'immunostimulant est ensuite stérilisé par filtration et lyophilisé.

**EXEMPLE 11**

Anticorps monoclonal-(MDP)$_{40}$ spécifique des cellules LLC.

Un anticorps monoclonal de souris spécifique des cellules du carcinome pulmonaire de Lewis (l'anticorps

IgM 6 B6) est dissous (4 mg/ml) dans un tampon acétate de sodium molaire pH 8,5. A cette solution on ajoute le MDP-O-Su décrit dans l'exemple 1b, puis la réaction est conduite comme dans l'exemple 1c. L'anticorps monoclonal-$(MDP)_{40}$ est purifié par tamisage moléculaire sur une colonne d'Ultrogel AcA 202. Les fractions contenant l'anticorps monoclonal-$(MDP)_{40}$ sont stérilisées par filtration sur Millipore (0,2 μ) puis conservées à 4°C.

On a réalisé la même expérience avec l'anticorps monoclonal 5B5.

**EXEMPLE 12**

Anticorps monoclonal-$(MDP)_{20}$ spécifique des cellules L1210.

Un anticorps monoclonal (IgM F2-10-23) de souris, spécifique des cellules de la leucémie L1210 est traité comme dans l'exemple 11.

**EXEMPLE 13**

Mannan-$(MDP)_{20}$.

Le mannane (50 mg) de Saccharomyces cerevisae est dissous dans 10 ml de diméthylsulfoxyde. A cette solution, on ajoute 10 mg de carbonyldiimidazole. Après 1 h à 4°C on ajoute 0,1 ml d'éthylène diamine. Après 20 h d'agitation à 4°C, le mannane substitué est purifié par tamisage moléculaire. Le mannane substitué est ensuite traité par le MDP-O-Su selon le protocole décrit dans l'exemple 1c.

Bien entendu, dans cet exemple, (MDP) est une notation abrégée dans laquelle on omet la mention de l'agent de couplages X qui représente ici $-CO-NH-C_2H_4-NH-$.

**EXEMPLE 14**

Néoglycoprotéine du type $(\alpha\text{-Man})_{25}$-SAB-$(MDP)_{18}$.

En opérant de façon analogue à celle décrite à l'exemple 1, on a préparé une néoglycoprotéine conjuguée du type $(\alpha\text{-Man})_{25}$-SAB-$(MDP)_{18}$. La notation $(\alpha\text{ -Man})_{25}$ est ici une abréviation pour $(\alpha\text{-Man-PTC})_{25}$.

**EXEMPLE 15**

Néoglycoprotéine du type $(6\text{-P-}\alpha\text{-Man})_{20}$-SAB-$(MDP)_{15}$.

De façon analogue à celle décrite à l'exemple 1, on a préparé cette néoglycoprotéine. (6-P-α-Man), qui signifie (6-phosphoryl-α-Man) est une notation abrégée pour (6-P-α-Man-PTC).

## ETUDE DES PROPRIETES PHARMACOLOGIQUES

### A) Activation de la tumoricidie des macrophages in vitro

#### 1) Cellules cibles

-L1210: cellules leucémiques de souris DBA/2 maintenue en culture, en suspension dans un milieu MEM contenant 10 % de sérum de cheval. Elles sont utilisées en phase exponentielle de croissance ($5{,}7 \times 10^5$ cell/ml).

- Cellules du carcinome pulmonaire de Lewis LLC

Elles proviennent d'une tumeur se développant spontanément dans les souris C57 BL6, et donnant de nombreuses métastases pulmonaires. Ces cellules sont cultivées en monocouches (ensemencées à $2 \times 10^4$ c/ml) dans un milieu de culture MEM contenant 20 % de sérum foetal de boeuf à 37°C et sous atmosphère de 5 % $CO_2$. A confluence (après 3 jours) elles sont récupérées par un bref traitement par une solution d'EDTA 0,02 % dans du tampon PBS contenant 0,1 % de glucose et reprises dans le milieu complet. Viabilité >95 %.

**2) Macrophages**

Macrophagés péritonéaux de souris $BDF_1$, appelés par 1 ml de milieu thioglycolate (Institut Pasteur). 4 jours après l'injection, les souris sont décapitées, et les cellules péritonéales récupérées par lavage avec le milieu de culture RPMI 1640 à 5 % de SVF (SVF: sérum foetal de veau). Après 2 lavages, les cellules sont mises en suspension dans le milieu RPMI 1640, 10 % SVF, contenant les antibiotiques et 0,1 % glutamine. On récupère 1-1,5 x $10^7$ cellules par souris dont 80-90 % sont des macrophages d'après la coloration au rouge neutre.

**3) Mesure de l'effet cytostatique**

En plaque de microtitration (Flacon Microtest II) 4 x $10^5$ macrophages incubés 4 h à 37° C (5 % $CO_2$); après 3 lavages pour éliminer les cellules non adhérentes, les macrophages sont incubés soit avec le stimulant (100 µl) (MDP, MDP couplé à une néoglycoprotéine ou à un polysaccharide ou à un anticorps polyclonal ou monoclonal), soit avec les solutions témoins correspondantes (protéine porteuse, PBS), dans RPMI 10 % SVF, antibiotique, glutamine, et 20 % tampon Hepes, 24 h à 37° C (5 % $CO_2$). Après 3 lavages, les cellules cibles sont ajoutées à différentes concentrations pour obtenir des rapports macrophage/cellules cibles variés (7 à 80). Après 20 h d'incubation on ajoute la thymidine tritiee (0,1 µ Ci 3,7 $KBq^3H$-thymidine, (CEA) 0,74-1,1 TBQ/mmole.

On peut également ajouter la thymidine en même temps que les cellules cibles. Les cellules sont récupérées sur filtre en fibre de verre, lavées à l'aide d'un appareil de type Mash II. On mesure la radioactivité des cellules dans un compteur β après dissolution dans un liquide scintillant (Tenu et al., 1980, Eur. J. Immunol. 10, 647-653).

Pourcentage d'inhibition $= \frac{R-S}{R} \times 100$

R = cellules cibles + macrophages non stimulés

S = cellules cibles + macrophages stimulés.

L'étude de l'activation des macrophages par les anticorps anti-cellules tumorales couplés au MDP (ou à ses dérivés) est réalisée en mettant simultanément en présence les macrophages, les cellules cibles et les stimulants. L'effet cytostatique est mesuré après 24-48 h de culture.

**4) Mesure de l'effet cytotoxique**

Il est mesuré par relargage de la radioactivité des cellules cibles qui ont été prémarquées avant d'être mises en présence des macrophages (Raz et al., 1979, Cancer Immunol. Immunother. 7, 157). Les cellules cibles en phase de croissance exponentielle sont marquées par incubation 24 h dans un milieu contenant 0,2 µ Ci (7,4 KBq)/ml d'($^{125}$I)IUdR c'est-à-dire de 5-($^{125}$I) Iodo-2'-déocyuridine Amersham 5 Ci/mg 185 GBq/mg. Les cellules sont lavées, et ajoutées aux macrophages stimulés et non stimulés. Les cellules cibles marquées sont également incubées seules comme témoins.

Après 24 h à 48 h, les cultures sont lavées, puis les cellules adhérentes sont lysées (0,1 ml 0,5 N NaCl) et lavées. Le lysat et les lavages sont rassemblés et la radioactivité mesurée dans un compteur gamma à scintillation. Le pourcentage de cytotoxicité médié par les macrophages activés est calculé.

$$\% \text{ cytotoxicité} = \frac{100 \text{ X Cpm (Mn) - Cpm (Ms)}}{\text{Cpm (Mn)} > t}$$

Cpm (Mn) signifie: nombre de coups par minute pour les cellules cibles cultivées avec macrophages normaux.

Cpm (Ms) signifie: nombre de coups par minute pour les cellules cibles cultivées avec macrophages stimulés.

Résultats: après 36 h, le MDP libre n'a aucun effet. Au contraire, le produit étudié $(Man)_{25}$-SAB-$(MDP)_{18}$ tue 50 % des cellules LLC ($^{125}$IUdR) à 1 µg/ml.

Remarque: les cellules LLC sont également appelées "3LL".

**5a) Application à la mesure de l'effet cytostatique des macrophages stimulés par le Man-SAB-MDP**

Le Man-SAB-MDP utilisé a 20 résidus de MDP par molécule de $(Man)_{25}$-SAB. Deux types de cellules cibles sont utilisés: les cellules leucémiques L1210 (figure 1) et les cellules du carcinome pulmonaire de Lewis, LLC (figure 2). Le MDP conjugué à la mannosyl-SAB rend les macrophages cytostatiques vis-à-vis des deux types de cellules tumorales utilisées. Le MDP conjugué est de surcroît plus actif in vitro que le MDP libre, il agit à des concentrations 10 fois plus faibles et augmente de façon très significative l'effet cytostatique des macrophages.

**TABLEAU I**

Activité cytostatique des macrophages sur les cellules L1210 et LLC.

| Traitement des macrophages | Macrophage<br>cellules cibles | % Cytostaticité<br>L1210 | LLC |
|---|---|---|---|
| MDP | 20 | 10 | 15 |
| | 40 | 12 | 20 |
| Man-SAB-MDP | 20 | 55 | 75 |
| | 40 | 70 | 85 |

4 x 10 macrophage sont stimulés par 1 µg/ml de MDP libre ou conjugué.

Les cellules cibles sont ajoutées après 24 h à différentes concentrations pour obtenir un rapport macrophage/cellules cibles de 20 et 40 et cocultivées 24 h. 4 heures avant la fin de la culture, 0,1 Ci, 3,7 Kbq de thymidine tritiée sont ajoutés. Le pourcentage de cytostaticité est calculé par rapport à la radioactivité incorporée par les cellules cibles cultivées en présence de macrophages non traités.

Dans les dessins annexés:

- la figure 1 représente l'effet cytostatique des macrophages péritonéaux de souris traitées par différentes doses de MDP libre ou conjugué à une néoglycoprotéine (Man-SAB) sur des cellules cibles L1210.

Rapport macrophage/L1210 = 20.

Le pourcentage de cytostaticité est la moyenne des valeurs de 3 cultures indépendantes, + 10 %;

- la figure 2 représente l'effet cytostatique des macrophages péritonéaux de souris traitées par différentes doses de MDP libre ou conjugué à une néoglycoprotéine (Man-SAB) sur des cellules LLC. Macrophage/LLC = 20;

- et la figure 3 représente l'effet cytostatique des macrophages péritonéaux de souris traitées par différentes doses de MDP libre ou conjugué à un anticorps monoclonal (IgM) dirigé contre la cellule cible L1210. Rapport Macrophage L1210=15.

Le pourcentage de cytostaticité est la moyenne de 3 cultures.

**5b) On a étudié de façon analogue le conjugué $(6\text{-P-}\alpha\text{-Man})_{20}\text{-SAB-}(MDP)_{15}$ de l'exemple 15.**

Ce composé a une affinité importante pour les monocytes qui sont ainsi fortement activés.

Il convient de remarquer que les monocytes fraîchement préparés à partir de sang circulant humain ne possèdent pas de récepteur pour le mannose et ne sont pas activés par les conjugués du type (α-Man)-SAB-(MDP)-

Par contre les monocytes humains reconnaissent le 6-phosphoryl mannose et, en présence de conjugé du type (6P-α-Man)-SAB-(MDP), ils sont rendus fortement cytostatiques et cytotoxiques vis-à-vis des cellules L1210 et des cellules du LLC.

**6) Application à la mesure de l'effet cytostatique des macrophages stimulés par le MDP conjugué à des anticorps monoclonaux** (figure 3)

- IgM anti L1210-MDP
- IgM anti LLC-MDP

Les callules sont respectivement les cellules leucémiques L1210 et les cellules du carcinome pulmonaire de Lewis (LLC) qui sont mises en présence de macrophage (Rapport macrophages/cellules cibles = 15) et des anticorps monoclonaux couplés au MDP. Après 36 h d'incubation, la $^{3}$H-thymidine est ajoutée à la culture. Les cellules sont récupérées et comptées aprés 4 h.

**7) Eradication des métastases spontanées du carcinome pulmonaire de Lewis dans des souris C57 B16 après amputation de la patte porteuse d'une tumeur primaire.**

Le principe du test est le suivant:

Des cellules tumorales du carcinome pulmonaire de Lewis (LLC) injectées dans la cuisse de souris syngéniques induisent la formation de tumeurs. Le développement des métastases augmente lorsque la tumeur est localement excisée chirurgicalement (Gorelik, E. et al. 21, 1980, 617-625); le volume des métaatases dépend de la quantité de cellules tumorales injectées et de la date de l'amputation (Gorelik, E. et al. (1980)

J.N.Cl. 65. 1257-1264).

6 x $10^6$ cellules LLC préparées par traitement à la trypsine d'une tumeur, sont injectées dans la cuisse d'un même lot de souris C57 B16.

Après 9 jours, la tumeur (diamètre: 1 cm) est éliminée par amputation haute de la patte au niveau de la cuisse après anesthésie. Au 12ème jour, le traitement est commencé: le MDP (4 µg) libre ou conjugué est injecté par voie intraveineuse à 3 ou 4 jours d'intervalle. A la fin du traitement, on measure le développement des métastases pulmonaires, par comptage de l'incorporation de la 5-iododéoxyuridine (ou $^{126}$IUdR incorporée); Bonmassar, E. et al., 1975, Chemotterapy 21, 321-329).

Il existe une relation entre l'incorporation de l'$^{125}$IUdR et l'étendue des tumeurs. Du fluorodéoxyuridine (25 µg/souris)est injecté par voie intrapéritonéale à chaque animal 30 minutes avant l'injection le µCi (37 KBq) d'$^{125}$IUdR afin d'inhiber la thymidilate synthétase et afin que les cellules incorporent préférentiellement IUdR. Après 24 h les souris sont sacrifiées, les poumons sont pesés et leur radioactivité est comptée à l'aide d'un γ-acintigraphe (LKB).

- 1 lot de 7 souris est traité par injection du conjugué $(Man)_{25}$-SAB-$(MDP)_{18}$ : lot A.
- 1 lot de 7 souris servant de témoin, est traité par injection d'une quantité équivalente de MDP et de Man-SAB, en mélange : lot B.
- 1 lot de 7 souris saines non traitées : lot C.

Toutes les souris du lot B témoin ont d'abondantes métastases pulmonaires, de 2 à 3 mm de diamètre, avec un début de nécrose. Parmi les souris traitées par le conjugué du type Man-SAB-MDP: une présente des métastases comparables à celles des témoins, une autre présente quelques petites métastases (<1 mm de diamètre) pulmonaire, les 5 autres souris ont des poumons d'apparence saine.

L'observation morphologique est configée par la mesure de l'incorporation de $^{125}$I IUdR (Tableau II). La moyenne est:

- 625 cpm/100 mg poumons, lot A, pour les 5 souris ayant un poumon d'apparence saine,
- 3100 cpm/100 mg poumons, lot B témoins. Poumons couverts de métastases. Dans ces mêmes conditions les souris saines, lot C, incorporent 800 cpm/ 100 mg poumons en moyenne.

En conclusion, 5 souris sur 7 ont été guéries par l'injection du complexe Man-BSA-MDP, alors que 7 souris sur 7 du lot témoin ont vu leurs métastases se développer abondamment comme en l'absence de tout traitement.

L'ablation de la tumeur (10 mm de diamètre) est effectuée au 12ème jour. Le traitement consiste en 3 injections intra-veineuses de 4 µg de MDP libre ou conjugué à 3 jours d'intervalle après l'amputation. Les métastases pulmonaires sont observées après sacrifice de l'animal, ou évaluées par incorporation de l'$^{125}$IUdR.

a) cpm: souris d'apparence saine (5/7)

b) cpm: souris avec métastases (2/7)

Une autre expérience analogue a donné les résultats suivants résumés dans le tableau II.

**TABLEAU II**

Mesure de l'incorporation de $^{125}$ I dans les métastases en coups par minute (Cpm)

| Traitement | Nombre de souris ayant: Plus de 20 métastases | | de 1 à 3 métastases | | 0 métastase | |
|---|---|---|---|---|---|---|
| | Nombre de souris | Cpm | Nombre de souris | Cpm | nombre de souris | Cpm |
| Témoins | 7/7 | 4 000 | 0/7 | - | 0/7 | - |
| Mélange MDP + (Man)-SAB | 7/7 | 3 700 | 0/7 | - | 0/7 | - |
| Conjugé (Man)-SAB-(MDP) | 1/7 | 8 000 | 3/7 | 400 | 3/7 | 0 |

**Revendications**

1. Nouveaux dérivés de formule générale I:

$(R\text{-}X)_m\text{-}(LAM)$ I

dans laquelle R est le résidu d'un immunostimulant du type N-acétylmuramylpeptide ou d'un de ses dérivés ou analogues,

(LAM) est un ligand affine des macrophages ou des monocytes, ou un précurseur d'un tel ligand,

et X représente soit une liaison covalente entre R et (LAM) soit le résidu d'un agent de couplage permettant de fixer R sur le ligand (LAM),

et m est un nombre supérieur ou égal à 1.

2. Nouveaux dérivés selon la revendication 1, caractérisés par le fait que R représente un résidu comportant

la séquence N-acétylmuramyl-L-Ala
et en particulier la séquence
N-acétylmuramy 1-L-Ala-D-isoGln-
ou N-acétylmuramyl-L-Ala-D-Glu-.

3. Dérivés selon la revendication 2, caractérisés par le fait que R est choisi parmi le résidu:
- du MDP (N-acétylmuramyl-L-Ala-D-isoGln)
- du MTP (N-acétylmuramyl-L-Ala-D-isoGln-meso-$A_2$pm)
- du MQP (N-acétylmuramyl-L-Ala-D-isoGln-meso-$A_2$pm-D-Ala)
- du MPP (N-acétylmuramyl-L-Ala-D-isoGln-meso-$A_2$pm-D-Ala-D-Ala)

ou un résidu muramyl peptidique substitué par un amino-acide, par un peptide, par un groupement bifonctionnel, par la glutaraldéhyde, ou par l'acide p-thiocarbamyl benzoïque.

4. Nouveaux dérivés selon l'une quelconque des revendications précédentes, caractérisés par le fait que le ligand affine des macrophages ou des monocytes est un glycoconjugué reconnu sélectivement par les lectines membranaires des macrophages ou des monocytes.

5. Dérivés selon la revendication 4, caractérisés par le fait que lesdits glycoconjugués sont choisis parmi les glycoprotéines naturelles ou modifiées, les néoglycoprotéines, les oligosaccharides ou les glycopeptides contenant au moins 5 motifs osidiques choisis parmi l'un au moins des résidus α-D-glycopyranoside, α-D-mannopyranoside, α-L-fucopyranoside, (α ou β)-L-galactopyranoside, β -D-galactopyranoside, 2-acétamido-2-déoxy (α ou β)-D-glucopyranoside, 2-acétamido-2-déoxy-α-D-galactopyranoside, 2-acétamido-2-déoxy-β-D-galactopyranoside, 6-phosphoryl-α-D-mannopyranoside ou α-L-rhamnopyranoside, ou les polysaccharides naturels ou modifiés portant des résidus α-D-glucose, α-D-mannose, α-L-fucose ou N-acétylglucosamine.

6. Dérivés selon la revendication 5, caractérisés par le fait que les motifs saccharidiques des oligosaccharides et/ou des glycopeptides sont partiellement estérifiés.

7. Dérivés selon la revendication 5, caractérisés par le fait que lesdites néoglycoprotéines sont des protéines syngéniques substituées par des copules glycidiques choisies parmi les osides de l'α-D-glucose, de l'α-D-mannose, de l'α-L-fucose, de la N-acétylglucosamine, de l'α-L-glucose du β-L-galactose, du β-D-galactose, de l'α-D-galactosamine, de la β-D-galactosamine, de l'α-L-rhamnose ou du 6-phosphoryl-α-D-mannose.

8. Dérivés selon la revendication 7, caractérisés par le fait que lesdites néoglycoprotéines comportent des osides choisis parmi:
- un résidu thiocarbamylphényl-α-D-glucopyranoside
- un résidu thiocarbamylphényl-β-D-galactopyranoside
- un résidu thiocarbamylphényl-α-D-mannopyranoside
- un résidu thiocarbamylphényl-2-acétamido-2-déoxy-β-D-glycopyranoside
- un résidu thiocarbamylphényl-α-L-fucopyranoside
- un résidu thiocarbamylphényl-α-D-galactopyranoside
- un résidu thiocarbamylphényl-α-L-rhamnopyranoside
- un résidu thiocarbamylphényl-6-phosphoryl α-D-mannopyranoside
- un résidu thiocarbamylphényl-2-acétamido-2-déoxy-α-D-glucopyranoside
- un résidu thiocarbamylphényl-2-acétamido-2-déoxy-α-D-galactopyranoside
- un résidu thiocarbamylphényl-2-acétamido-2-déoxy-α-D-galactopyranoside
- un résidu thiocarbamylphényl-β-D-galacto-pyranosyl-4-β-D-glucopyranoside
- un résidu azophényl-β-D-galactopyranosyl-4-β-D-glucopyranoside
- un résidu azophényl-α-D-glucopyranoside
- un résidu azophényl-α-D-mannopyranoside
- un résidu azophényl-α-L-fucopyranoside
- un résidu azophényl-β-D-galactopyranoside
- un résidu azophényl-2-acétamido-2-déoxy-β-D-glycopyranoside
- les résidus d'oligosaccharides ou de glycopeptides renfermant l'un au moins des résidus α-D-glucopyranoside, α-L-glucopyranoside, α-D-mannopyranoside, α-L-fucopyranoside, β-L-galactopyranoside, β-D-galactopyranoside, 2-acétamido-2-déoxy-β-D-glucopyranoside, 2-acétamido-2-déoxy-α-D-galacto- pyrano side, 2-acétamido-2-déoxy-β-D-galactopyranoside, 6-phosphoryl-α-D-man-nopyrano side, α-L-rhamnopyranoside.

9. Dérivés selon la revention 7 ou 8, caractérisés par le fait que ladite protéine est la sérumalbumine, le lysozyme, la ferritine ou une immunoglobuline.

10. Dérivés selon la revendication 5, caractérisés par le fait que lesdits polysaccharides sont choisis parmi les polysaccharides de levures, en particulier le mannane, les polysaccharides de bactéries, ou encore d'autres polysaccharides comme la fucoïdine ou le lentinane.

11. Dérivés selon l'une quelconque des revendications 1 à 3, caractérisés par le fait que le ligand affine des macrophages ou des monocytes est constitué par une protéine syngénique modifiée par des substitutions abaissant le point isoélectrique.

12. Dérivés selon la revendication 11, caractérisés par le fait que lesdites protéines modifiées sont des protéines N-acylées ou des protéines S-carboxyméthylées.

13. Dérivés selon l'une quelconque des revendications 1 à 3, caractérisés par le fait que ledit précurseur de ligand affine des macrophages ou des monocytes est une antiprotéase.

14. Dérivés selon la revendication 13, caractérisés par le fait que l'antiprotéase est choisi parmi les

antiprotéases sériques syngénique telles que 1'$\alpha_2$-macroglobuline, 1'$\alpha_1$-antitrypsine, ou 1'$\alpha_1$-antichimotrypsine.

15. Nouveaux dérivés selon l'une quelconque des revendications 1 à 3, caractérisés par le fait que ledit précurseur de ligand affine de macrophages est un anticorps anti-cellules tumorales.

16. Dérivés selon la revendication 15, caractérisés par le fait que ledit anticorps est un anticorps monoclonal.

17. Procédé de préparation des dérivés selon l'une quelconque des revendications précédentes, caractérisé par le fait que l'on fait réagir selon les méthodes connues le dérivé (R) correspondant au résidu R avec le ligand (LAM), éventuellement en présence d'un agent de couplage.

18. Utilisation pour la préparation d'un médicament, notamment immunostimulant, des nouveaux dérivés tels que définis dans l'une quelconque des revendications 1 à 16.

19. Utilisation selon la revendication 18, pour la préparation d'un médicament stimulant l'activité des monocytes et des macrophages, notamment l'action tumoricide des macrophages.

20. Utilisation selon la revendication 18, pour la préparation d'un médicament contre le développement des cellules tumorales, y compris des cellules de métastases spontanées.

21. Utilisation selon l'une quelconque des revendications 18 à 20, caractérisée par le fait que lesdits dérivés sont tels que définis dans l'une quelconque des revendications 7 à 9.

22. Compositions pharmaceutiques, caractérisées par le fait qu'elles contiennent au moins un dérivé de formule I tel que défini dans l'une quelconque des revendications 1 à 16.

23. Compositions selon la revendication 22, caractérisées par le fait que ledit dérivé de formule I est tel que défini dans l'une quelconque des revendications 7 à 9.

**Patentansprüche**

1. Neue Derivate der allgemeinen Formel I:

$(R\text{-}X)_m$ - (LAM) I

worin

R den Rest eines Immunostimulans vom Typ N-Acétylmuramylpeptid oder eines anologen Derivats davon ist,

LAM ein Ligand ist mit einer Affinität zu Macrophagen oder zu Monocyten, oder ein Vorläufer eines derartigen Liganden darstellt, und

X entweder eine covalente Bindung zwischen R und (LAM) oder den Rest eines Kupplungsmittels, das die Fixierung von R an den Liganden (LAM) gestattet, darstellt

m eine Zahl größer oder = 1 ist.

2. Neue Derivate nach Anspruch 1, dadurch gekennzeichnet, dass R einen Rest der Sequenz N-Acétylmuramyl-L-Ala- und insbesondere der Sequenz

N-Acétylmuramyl-L-Ala-D-isoGln- oder

N-Acétylmuramyl-L-Ala-D-Glu- bedeutet.

3. Derivate nach Anspruch 2, dadurch gekennzeichnet, dass R ausgewählt ist auf den Resten

MDP (N-Acétylmuramyl-L-Ala-D-isoGln)

MTP (N-Acétylmuramyl-L-Ala-D-isoGln-meso-$A_2$ pm)

MQP (N-Acétylmuramyl-L-Ala-D-isoGln-meso-$A_2$ pm-D-Ala)

MPP (N-Acétylmuramyl-L-Ala-D-isoGln-meso-$A_2$ pm-D-Ala-D-Ala)

oder einen Muramylpeptidrest darstellt, der durch eine Aminosäure, eine Peptid, ein bi-funktionelle Gruppe, Glutaraldehyd oder durch p-Thiocarbamylbenzoesäure substitiuiert ist, darstellt.

4. Neue Derivate nach einem der vorhergehenden Ansprüche, dadurch gekennzeichnet, dass der Ligand mit der Affinität zu Macrophagen oder Monocyten eine selektiv für Membranlectine von Macrophagen oder Monocyten bekannte glycoconjugierte Substanz ist.

5. Derivate nach Anspruch 4, dadurch geknnzeichnet, dass die glycoconjugierten Substanzen ausgewählt sind aus natürlichen oder modifizierten Glycoproteinen, Neoglycoproteinen, Oligosacchariden, oder Glycopeptiden, die mindestens 5 Osidbestandteile enthalten, ausgewählt aus mindestens einem der Reste α-D-Glycopyranosid, α-D-Mannopyranosid, α-L-Fucopyranosid, (α oder β)-L-Galactopyranosid, β-D-Galactopyranosid, 2-Acetamido-2-Deoxy (α oder β)-D-Glucopyranosid, 2-Acetamido-2-deoxy-α-D-galactopyranosid, 2-Acetamido-2-deoxy-β-D-galactopyranosid, 6-Phosphoryl-α-D-mannopyranoside oder α-L-Rhamnopyranosid oder der natürlichen oder modifizierten Polysaccharide, welche die Reste von α-D-Glucose, α-D-Mannose, α-L-Fucose oder N-Acétylglucosamin tragen.

6. Derivate nach Anspruch 5, dadurch gekennzeichnet, dass die Saccharidbestandteile der Oligosacharine und/oder der Glycopeptide teilweise verestert sind.

7. Derivate nach Anspruch 5, dadurch gekennzeichnet, dass die genannten Neoglycoproteine syngenische Proteine sind, die durch Glycid-Haftstellen substituiert sind, welche ausgewählt sind aus Osiden von α-D-Glucose, α-D-Mannose, α-L-Fucose, N-Acétylglucosamin, α-L-Glucose, β-L-Galactose, β-D-Galactose, -D-Galactosamin, β-D-Galactosamin, α-L-Rhamnose oder 6-Phosphoryl-α-D-Mannose.

8. Derivate nach Anspruch 7, dadurch gekennzeichnet, dass die Neoglycoproteine Oside tragen, die ausgewählt sind aus folgenden Resten

dem Thiocarbamylphenyl-α-D-glucopyranosidrest
dem Thiocarbamylphenyl-β-D-galactopyranosidrest
dem Thiocarbamylphenyl-α-D-mannopyranosidrest
dem Thiocarbamylphenyl-2-Acetamido-2-deoxy-β-D-glycopyranosidrest
dem Thiocarbamylphenyl-α-L-fucopyranosidrest
dem Thiocarbamylphenyl-α-D-galactopyranosidrest
dem Thiocarbamylphenyl-α-L-rhamnopyranosidrest
dem Thiocarbamylphenyl-6-phosphoryl α-D-mannopyranosidrest
dem Thiocarbamylphenyl-2-acetamido-2-deoxy-α-D-glucopyranosidrest
dem Thiocarbamylphenyl-2-acetamido-2-deoxy-β-D-galactopyranosidrest
dem Thiocarbamylphenyl-2-acetamido-2-deoxy-β-D-galactopy-ranosidrest
dem Azophenyl-β-D-galactopyranosil-4-β-D-glucopyranosidrest
dem Azophenyl-β-D-glucopyranosidrest
dem Azophenyl-α-D-mannopyranosidrest
dem Azophenyl-α-L-fucopyranosidrest
dem Azophenyl-β-D-galactopyranosidrest
dem Azophenyl-2-acétamido-2-deoxy-β-D-glycopyranosidrest
den Oligosaccharidresten oder Glycopeptidresten,
die mindestens einen der folgenden Resten enthalten:
α-D-Glucopyranosid, α-L-Glucopyranosid, α-D-Mannopyranosid, α-L-Fucopyranosid, β-L-Galactopyranosid, β-D-Galactopyranosid, 2-Acétamido-2-deoxy-β-D-glucopyranosid, 2-Acétamido-2-deoxy-α-D-galactopyranosid, 2-Acétamido-2-deoxy-β-D-galactopyranosid, 6-Phosphoryl-α-D-mannopyranosid, α-L-Rhamnopyranosid.

9. Derivate nach Anspruch 7 oder 8, dadurch gekennzeichnet, dass das Protein ein Serumalbumin, Lysozym, ein Ferritin oder ein Immunoglobulin darstellt.

10. Derivate nach Anspruch 5, dadurch gekennzeichnet, dass die Polysaccharide ausgewählt sind aus Polysacchariden von Hefen, insbesondere von Weinhefen, Polysaccharide von Bakterien oder noch andere Polysaccharide wie Fucoidin oder Lentinan.

11. Derivate nach einem der Ansprüche 1 bis 3, dadurch gekennzeichnet, dass der Ligand mit Affinität zu Macrophagen oder Monocyten aus einem syngenischen Protein besteht, das durch Substitutionen unterhalb des isoelektrischen Punktes modifiziert ist.

12. Derivate nach Anspruch 11, dadurch gekennzeichnet, dass die modifizierte Proteine N-acylierte Proteine oder S-carboxymethylierte Proteine sind.

13. Derivate nach einem der Ansprüche 1 bis 3, dadurch gekennzeichnet, dass der Vorläufer des Liganden mit der Affinität zu Macrophagen oder Monocyten eine Antiprotease ist.

14. Derivate nach Anspruch 13, dadurch gekennzeichnet, dass die Antiprotease ausgewählt ist aus serisch syngenischen Antiproteasen, wie $\alpha_2$-Macroglo-bulin, $\alpha_1$-Antitrypsin oder $\alpha_1$-Antichimotrypsin.

15. Neue Derivate nach einem der Ansprüche 1 bis 3, dadurch gekennzeichnet, dass die Vorläufer des Liganden mit Affinität zum Macrophagen ein anti-Tumorzellen-antikörper ist.

16. Derivate nach Anspruch 15, dadurch gekennzeichnet, dass die Anti-körper monoklonale Anti-körper sind.

17. Verfahren zur Herstellung von Derivaten nach einem der vorherigen Ansprüche, dadurch gekennzeichnet, dass man nach bekannten Verfahren das Derivat R entsprechend dem Rest R mit dem Liganden (LAM) gegebenenfalls in Gegenwart eines Kupplungsmittels reagieren läßt.

18. Verwendung der neuen Derivate nach der Definition in einem der Ansprüche 1 bis 16 als Arzneimittel, insbesondere Immunostimulanz.

19. Verwendung nach Anspruch 18 zur Herstellung eines Arzneimittels, das die Aktivitäten von Monocyten und Macrophagen stimuliert, insbesondere die Tumorizidwirkung von Macrophagen.

20. Verwendung nach Anspruch 18 zur Herstellung eines Arzneimittels gegen die Entwicklung von Tumorzellen und insbesondere von spontanen Metastasenzellen.

21. Verwendung nach einem der Ansprüche 18 bis 20, dadurch gekennzeichnet, dass die Derivate solche sind, wie sie in einem der Ansprüche 7 bis 9 definiert sind.

22. Arzneimittelzubereitungen, dadurch gekennzeichnet, dass sie mindestens ein Derivat der Formel I gemäß der Definitionen in einem der Ansprüche 1 bis 16 enthalten.

23. Zubereitungen nach Anspruch 22, dadurch gekennzeichnet, dass die Derivate der Formel I solche sind, wie sie in einem der Ansprüche 7 bis 9 definiert sind.

**Claims**

1. New derivatives of general formula I:
$(R\text{-}X)_m\text{-}(LAM)$ I
in which R is the residue of in immunostimulant of the N- acetylmuramyl peptide type or of one of its derivatives or analogues,
(LAM) is a ligand having affinity for macrophages or monocytes, or a precursor of such a ligand,
and X denotes either B covalent bond between R and (LAM) or the residue of a coupling agent enabling R to

be attachid to the ligand (LAM),

and m is a number greater than of equal to 1.

2. New derivitives according to Claim 1, characterized in that R denotes a residue containing the sequence N-acetylmuramyl-L-Alb-

and in particular the sequence

N-acetylmuramyl-L-Ala-D-isoGln-

or N-acetylmuramyl-L-Ala-D-Glu-.

3. Derivatives according to Claim 2, characterised in that R is chosen from the residue:

of MDP (N-acetylmurnmyl-L-Ala-D-isoGLn)

of MTP (N-acetylmurhmyl-L-Ala-D-isoGln-meso-$A_2$pm)

of MQP (N-acetylmuramyl-L-Ala-D-isoGln-meso-$A_2$pm-D-Ala)

of MPP (N-acetylmuramyl-L-Ala-D-isoGln-meso-$A_2$pm-D-Ala-D-Ala)

or a muramyl peptide residue substituted with an amino acid, with a peptide, with a bilfunctional group, with glutaraldehyde or with p-thiocarbamylbenzoic acid.

4. New derivatives according to any one of the preceding claims, characterized in that the ligand having affinity for macrophages or monocytes is a glyco-conjugate, recognixed selectively by the membrane lections of macrophages or monocytes.

5. Derivatives according to Claim 4, characterized in that the said glyco-conjugates are chosen from natural or modified glycoproteins, neoglycoproteins, oligosaccharides or glycopeptides containing at least 5 glycoside units chosen from at least one of the residues α-D-glycopyranoside, α-D-mannopyranoside, α-L-fucopyranoside, (α or β)-L-galactopyranoside, β-D-galactopyranoside, 2-acetamido-2-deoxy(α or β)-D-glucopyranoside, 2-acetamido-2-deoxy-α-D-galactopyranoside, 2-acetimido-2-deoxy-β-D-galactopyranoside, 6-phosphoryl-α-D-mannopyranoside or α-L-rhamnopyranoside, or natural or modified polysaccharides bearing α-D-glucose, α-D-mannose, α-L-fucose or N- acetylglucosamine residues.

6. Derivatives according to Claim 5, characterised in that the saccharide units of the oligosiaccharides and/or glycopeptides are partially esterfied.

7. Derivitives according to Claim 5, characterized in that the said neoglycoproteins are syngenic proteins substituted with sugar linkages chosen from the glycosides of α-D-glucose, α-D-mannose, α-L-fucose, N-acetylglucosamine, α-L-glucose, β-L-galactose, β-D-galactose, α-D-galactosamine, β-D-galactosamine, α-L-rhamnose or 6-phosphoryl-α-D-mannose.

8. Derivatives according to Claim 7, characterized in that the said neoglycoproteins contain glycosides chosen from:

a thiocarbamylphenyl-α-D-glucopyranoside residue

a thiocarbamylphenyl-β-D-galactopyranoside residue

a thiocarbamylphenyl-α-D-mannopyranoside residue

a thiocarbamylphenyl-2-acetamido-2-deoxy-β-D-glycopyranoside residue

a thiocarbamylphenyl-α-L-fucopyranoside residue

a thiocarbamylphenyl-α-D-galactopyranoside residue

a thiocarbamylphenyl-α-L-rhamnopyranoside residue

a thiocarbamylphenyl-6-phosphoryl-α-D-mannopyranoside residue

a thiocarbamylphenyl-2-acetamido-2-deoxy-α-D-glucopyranoside residue

a thiocarbamylphenyl-2-acetamido-2-deoxy-α-D-galactopyranoside residue

a thiocarbamylphenyl-2-acetamido-2-deoxy-α-D-galactopyranoside residue

a thiocarbamylphenyl-β-D-galactopyranosyl-4-β-D-glucopyranoside residue

an azophenyl-β-D-galactopyranosyl-4-β-D-glycopyranoside residue

an azophenyl-α-D-glucopyranoside residue

an azophenyl-α-D-mannopyranoside residue

an azophenyl-α-L-fucopyranoside residue

an azophenyl-β-D-galactopyranoside residue

an azophenyl-2-acetamido-2-deoxy-β-D-glycopyranoside residue

the residues of oligoseccharides or glycopeptides containing at least one of the residues α-D-glucopyranoside, α-L-glycopyranoside, α-D-mannopyranoside, α-L-fucopyranoside, β-L-galactopyranoside, β-D-galactopyranoside, 2-acetamido-2-deoxy-β-D-glucopyranoside, 2-acetamido-2-deoxy-α-D-galactopyranoside, 2-acetamido-2-deoxy-β-D-galactopyranoside, 6-phosphoryl-α-D-mannopyranoside and α-L-rhamnophyrannoside.

9. Dervivatives according to Claim 7 or 8, characterized in that the said protein is serum albumin, lysozyme, ferritin or an immunoglobulin.

10. Derivatives according to Claim 5, characterized in that the said polysaccharides are chosen from yeast polysaccharides, especially mannan, bacterial polysaccharides or alternatively other polysaccharides such as fucoidin or lentinan.

11. Derivatives according to any one of Claims 1 to 3, characterized in that the ligand having affinity for macrophages or monocytes consists of a syngenic protein modified by substitutions which lower the isoelectric point.

12. Derivatives according to Claim 11, characterized in that the said modified proteins are N-acyl proteins or S-carboxymethyl proteins.

13. Derivatives according to any one of Claims 1 to 3, characterized in that the said precursor of a ligand having affinity for macrophages or monocytes is an antiprotease.

14. Derivatives according to Claim 13, characterized in that the antiprotease is chosen from syngenic serum antiproteeses such as $\alpha_2$-marcoglobulin, $\alpha_1$-antitrypsin or $\alpha_1$-antichymotrypsin.

15. New derivatives according to any one of Claims 1 to 3, characterized in that the said precursor of a ligand having affinity for macrophages is an antibody against tumour cells.

16. Derivatives according to Claim 15, characterized in that the said antibody is a monoclonal antibody.

17. Method for preparing derivatives according to any one of the preceding claims, characterized in that the derivative (R) corresponding to the residue R is reacted according to known methods with the ligand (LAM), where appropriate in the presence of a coupling agent.

18. Use for the preparation of a medicinal product, in particular an immunostimulant, of the new derivatives as defined in any one of Claims 1 to 16.

19. Use according to Claim 18, for the preparation of a medicinal product stimulating monocyte and macrophage activity, in particular the tumoricidal action of macrophages.

20. Use according to Claim 18, for the preparation of a medicinal product counteracting the development of tumour cells, including cells of spontaneous metastases.

21. Use according to any one of Claims 18 to 20, characterized in that the said derivatives are as defined in any one of Claims 7 to 9.

22. Pharmaceutical compositions, characterized in that they contain at least one derivative of formula I as defined in any one of Claims 1 to 16.

23. Compositions according to Claim 22, charactirized in that the said derivative of formula I is as defined in any of Claims 7 to 9.

1/3

Fig. 1

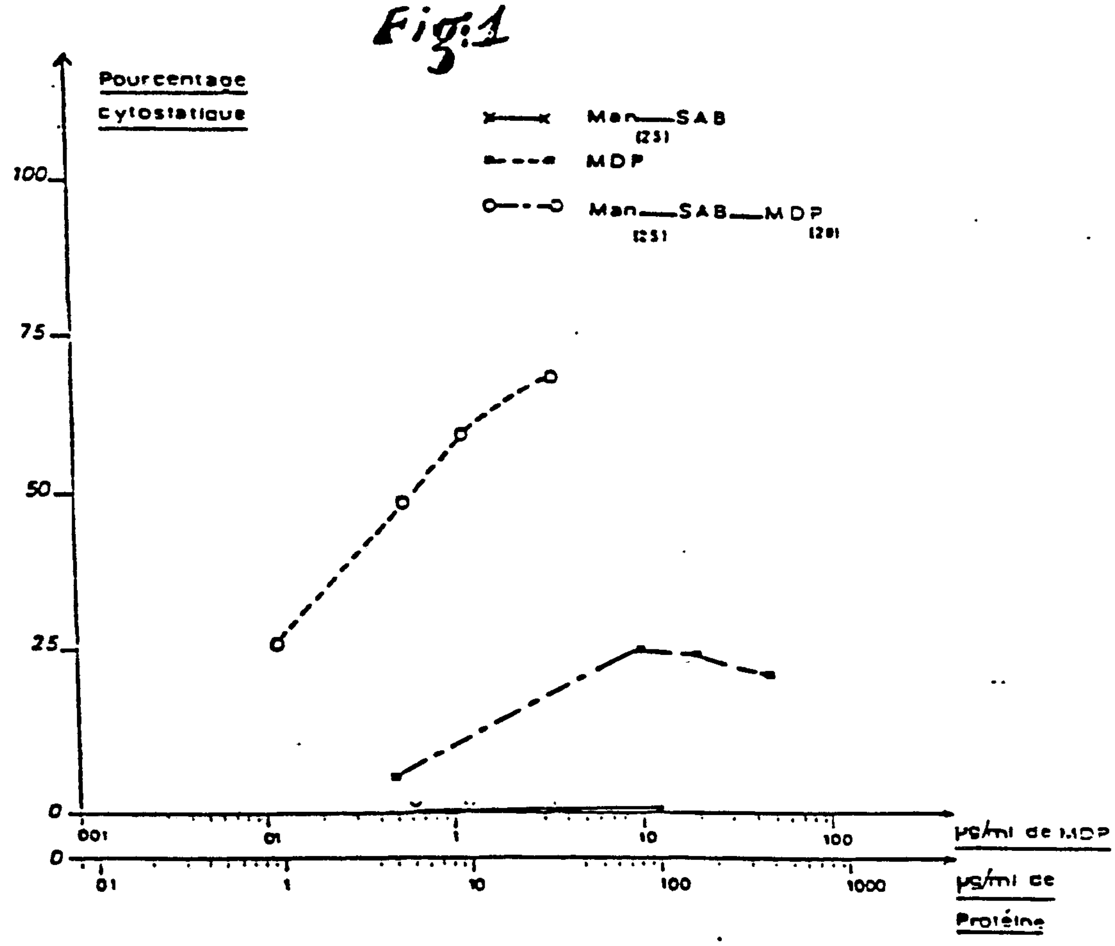
Pourcentage cytostatique
Man—SAB (25)
MDP
Man—SAB—MDP (25) (28)
100
75
50
25
0
001
01
1
10
100
µg/ml de MDP
0
01
1
10
100
1000
µg/ml de Protéine

Fig: 2

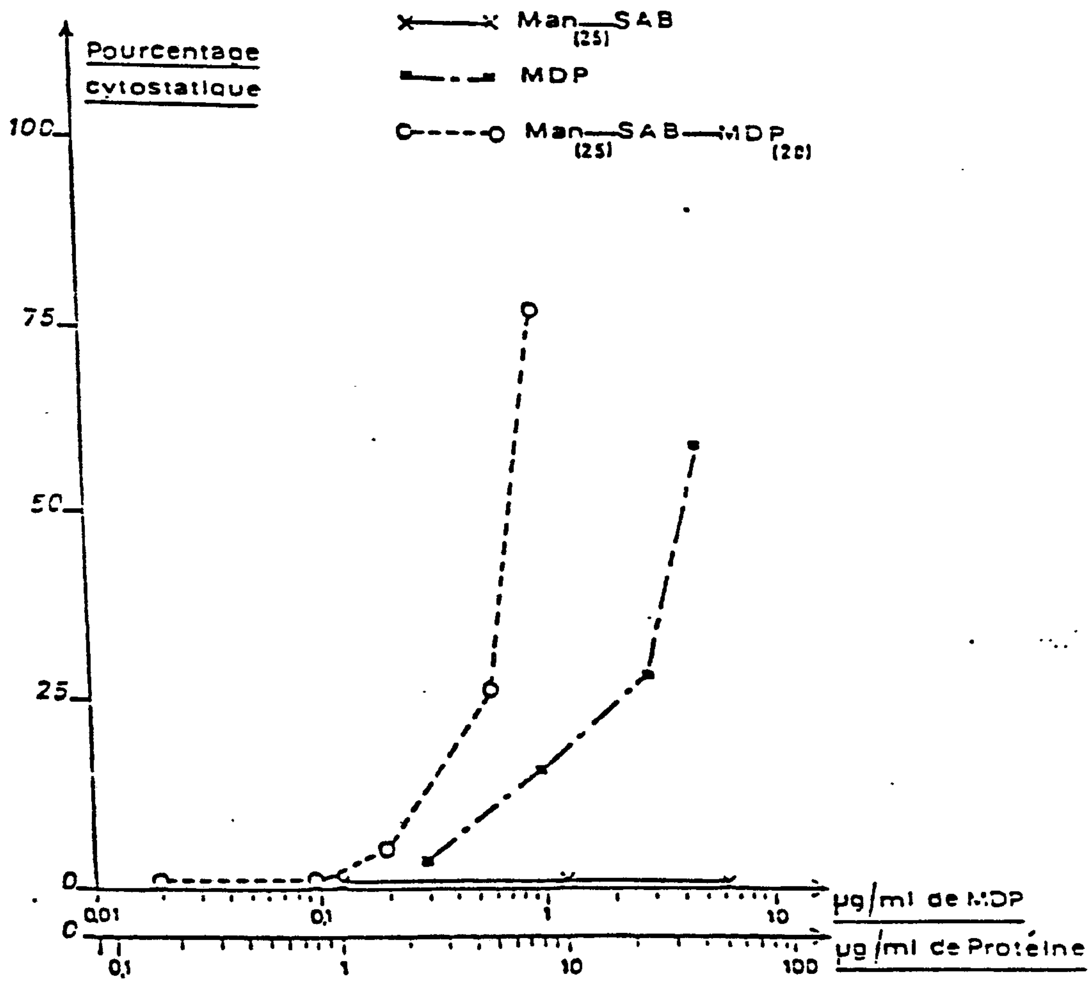
Man(25)—SAB
MDP
Man(25)—SAB—MDP(20)
Pourcentage cytostatique
100
75
50
25
0
0,01
0,1
1
10
µg/ml de MDP
0,1
1
10
100
µg/ml de Protéine

Fig: 3

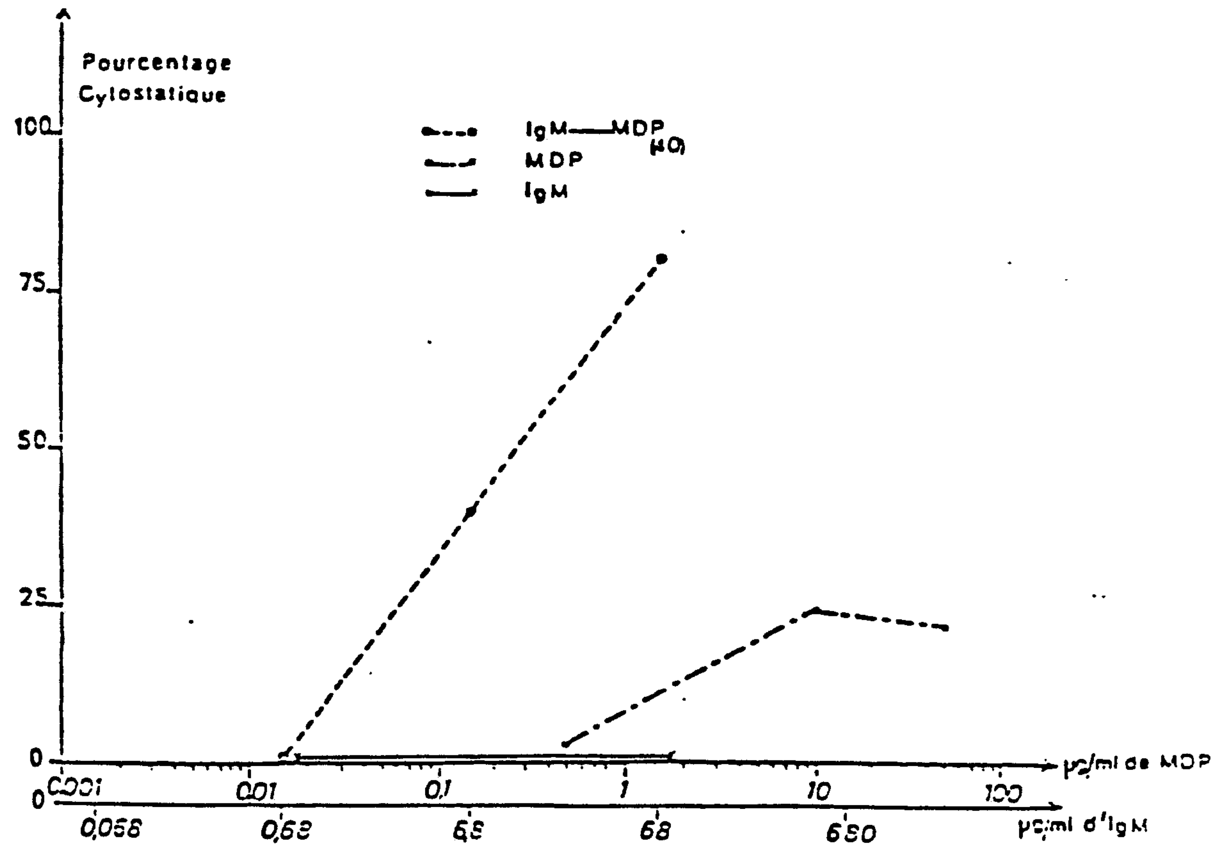
Pourcentage
Cytostatique
100
75
50
25
0
IgM——MDP (μO)
MDP
IgM
0,001
0,01
0,1
1
10
100
μg/ml de MDP
0
0,068
0,68
6,8
68
680
μg/ml d'IgM